# EUROPEAN PATENT SPECIFICATION

(11) **EP 2 236 164 B1**
(45) Date of publication and mention of the grant of the patent: **07.05.2014**
(21) Application number: 10250665.6
(22) Date of filing: 30.03.2010
(51) Int. Cl.: A61M 5/142

(54) **Insulin cartridge cap**
Insulinkartuschenkappe
Fermeture de cartouche d'insuline

(30) Priority: 31.03.2009 US 165038 P
(43) Date of publication of application: 06.10.2010
(73) Proprietor: Animas Corporation, West Chester, PA 19380 (US)
(72) Inventor: Myland, Lawrence, West Chester, PA 19380 (US)
(74) Representative: Tunstall, Christopher Stephen

(56) References cited:
- EP-A1- 1 985 322
- DE-A1- 10 146 454
- US-A1- 2003 163 090

## Description

### FIELD OF THE INVENTION

The present invention relates, in general, to cartridge caps used in drug delivery devices and, more particularly, to cartridge caps with a feature for removing a battery cap.

### BACKGROUND OF THE INVENTION

The use of drug delivery devices for various types of drug therapy is becoming more common as the automated infusion of a drug may provide more reliable and more precise treatment to a patient.

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia which, if left uncorrected, can lead to ketosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

In order to more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid which is then analyzed by the meter. In most cases, the meter has a display screen which shows the BG reading for the patient. The patient may then dose themselves with the appropriate amount, or bolus, of insulin. For many diabetics, this results in having to receive multiple daily injections of insulin. In many cases, these injections are self-administered.

Due to the debilitating effects that abnormal BG levels can have on patients, i.e., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed.

Insulin pumps are generally worn on the patient's body, either above or below their clothing. These relatively small, unobtrusive devices typically store a quantity of insulin in a replaceable cartridge and include a processing unit, a display screen, and input functions such as buttons or a keypad. Such pumps may include the ability to run multiple insulin delivery programs, such as basal and bolus programs, to eliminate the need for injections of insulin via needles and syringes, by providing medication via an infusion device that can be worn by the patient for an extended period of time, usually in the range of 1-3 days.

An example of prior art devices is given in US 2003/163 090 A1.

While the convenience of an insulin pump has helped to improve the lifestyle of diabetics and has lessened the impact of their disease on their normal activity, advances in insulin pumps are still needed. For example, when the battery needs to be replace, the user must find a tool to remove the battery cap. Typically, users use a coin to open the battery cap. However, using a coin to open the battery cap may be difficult for users with arthritis or weakened motor skills.

Therefore, it would be desirable for patients to have a tool that facilitates easy removal of the battery cap and that is a component of the insulin pump.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 is a perspective view of an insulin cartridge cap and a battery cap according to an exemplary embodiment of the present invention;

FIG. 2 is a perspective view of an insulin pump that may be used with the cartridge cap and battery cap shown in FIG. 1;

FIG. 3 is a perspective view of the cartridge cap shown in FIG. 1 being used to remove the battery cap shown in FIG. 1 from an insulin pump; and

FIG. 4 is a top view of the battery cap shown in FIG. 1.

### DETAILED DESCRIPTION OF ILLUSTRATIVE EMBODIMENTS OF THE INVENTION

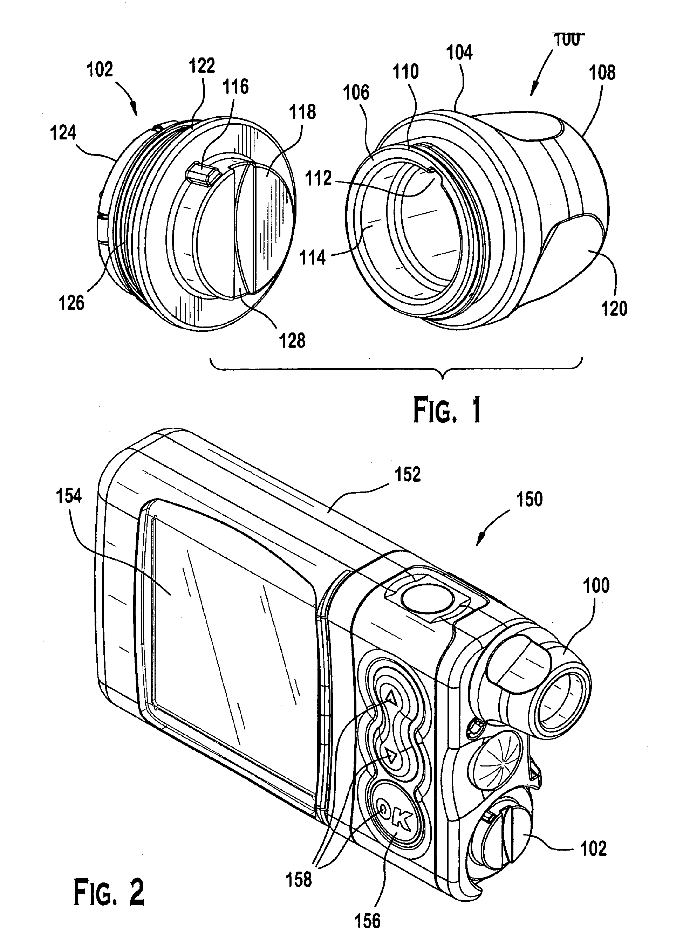

FIG. 1 illustrates an insulin cartridge cap 100 and a battery cap 102 according to an exemplary embodiment of the present invention. The cartridge cap 100 includes a body 104 having a proximal end 106 and a distal end 108. The proximal end 106 includes threads 110 that mate with threads in an insulin cartridge chamber (not shown) of a drug infusion pump 150 (shown in FIG. 2). The proximal end 106 also includes a recess 112 on an inner surface 114 that receives a tab 116 on a distal end 118 of the battery cap 102.

The distal end 108 of the cartridge cap 100 may include indentations 120 to aid in gripping the cartridge cap 100.

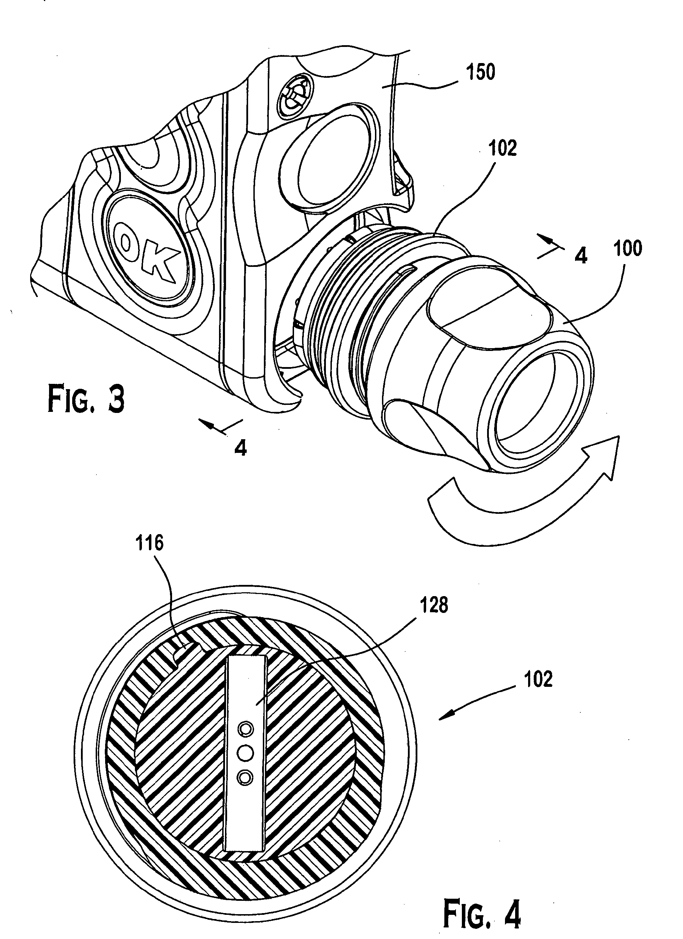

The battery cap 102 includes a body 122, the distal end 118 and a proximal end 124. The proximal end 124 includes threads 126 that mate with threads in a battery chamber (not shown) of the drug infusion pump 150. The distal end may 118 optionally include a groove 128 into which a coin can be inserted for removal of the battery cap 102 (see FIGS. 1 and 4).

An exemplary embodiment of a drug infusion pump 150 (e.g., an insulin pump) that may incorporate the cartridge cap 100 and the battery cap 102 of the present invention is illustrated in FIG. 2. The drug infusion pump 150 includes a housing 152, a display 154 for providing operational information to the user, a keypad 156 with a plurality of navigational buttons 158 for the user to input information, a battery in a compartment (not shown) with a battery cap 102 for providing power to the drug infusion pump 150, processing electronics (not shown), a drug delivery mechanism (e.g., an insulin pump and drive mechanism; not shown) for forcing a drug from a cartridge in a chamber with a cartridge cap 100, through a side port (not shown) connected to an infusion set (not shown) and into the body of the user.

To use the cartridge cap 100 as a tool to remove the battery cap 102, the cartridge cap 100 is removed from the insulin cartridge chamber of the infusion pump and is placed on the battery cap 102. The cartridge cap 100 is then rotated either clockwise or counterclockwise until the recess 112 is aligned and mated with the tab 116 on the battery cap 102. As shown in FIG. 3, after the recess 112 is mated with the tab 116, the cartridge cap 100 is rotated counterclockwise until the battery cap 102 is removed.

It should be understood that various alternatives to the embodiments of the invention described herein may be employed in practicing the invention. It is intended that the following claim defines the scope of the invention and that methods and structures within the scope of this claim and their equivalents be covered thereby.

## Claims

1. An medical device (150), comprising:
a housing (152) having a cartridge cavity therein:
a cartridge capable of containing a quantity of fluid, wherein the cartridge cavity receives the cartridge;
a cartridge cap (100) that is removable attachable to the cartridge cavity;
a battery cavity for receiving a battery;
a battery cap (102) that is removably attachable to the battery cavity, and
wherein the battery cap (102) and cartridge cap (100) each have at least one interlocking feature (112, 116) to permit the battery cap (102) and cartridge cap (100) to interlock and for the cartridge cap (100) to be used as a tool to remove or affix the battery cap (102) to the battery cavity.

## Patentansprüche

1. Medizinische Vorrichtung (150), umfassend:
ein Gehäuse (152) mit einem darin angeordneten Patronenhohlraum,
eine Patrone, die eine Fluidmenge enthalten kann,
wobei der Patronenhohlraum die Patrone aufnimmt,
eine Patronenkappe (100), die entfernbar am Patronenhohlraum anbringbar ist,
einen Batteriehohlraum zur Aufnahme einer Batterie,
eine Batteriekappe (102), die entfernbar am Batteriehohlraum anbringbar ist, und
wobei die Batteriekappe (102) und die Patronenkappe (100) jeweils mindestens ein Verriegelungsmerkmal (112, 116) haben, um eine Verriegelung der Batteriekappe (102) und der Patronenkappe (100) zu gestatten und damit die Patronenkappe (100) als ein Werkzeug verwendet werden kann, um die Batteriekappe (102) aus dem Batteriehohlraum zu entfernen oder dort zu befestigen.

## Revendications

1. Dispositif médical (150) comprenant :
un boîtier (152) comprenant une cavité de cartouche ;
une cartouche susceptible de contenir une quantité de liquide, la cavité de cartouche recevant la cartouche ;
un capuchon de cartouche (100) qui peut être fixé de manière amovible à la cavité de cartouche ;
une cavité de pile pour recevoir une pile ;
un capuchon de pile (102) qui peut être fixé de manière amovible à la cavité de pile et
le capuchon de pile (102) et le capuchon de cartouche (100) ayant chacun au moins un élément de verrouillage réciproque (112, 116) pour permettre au capuchon de pile (102) et au capuchon de cartouche (100) de se verrouiller réciproquement et pour que le capuchon de cartouche (100) soit utilisé en tant qu'outil pour retirer le capuchon de pile (102) de la cavité de pile ou pour l'attacher à cette dernière.
